# EUROPÄISCHE PATENTANMELDUNG

(11) **EP 4 477 964 A1**
(43) Veröffentlichungstag der Anmeldung: **18.12.2024**
(21) Anmeldenummer: 24175122.1
(22) Anmeldetag: 10.05.2024
(51) Int. Cl.: F24F 8/30, A61L 9/22

(54) **VORRICHTUNG ZUR IONISIERUNG VON LUFT SOWIE STRÖMUNGSKANAL**

(30) Priorität: 24.05.2023 DE 102023113558
(71) Anmelder: Vientum GmbH, 79346 Endingen am Kaiserstuhl (DE)
(72) Erfinder: Weigele, Uwe Dieter, 88605 Meßkirch (DE); Rieder, Erhard, 71083 Herrenberg (DE)
(74) Vertreter: Westphal, Mussgnug & Partner Patentanwälte mbB

(57) **Zusammenfassung**

Vorrichtung (10) zur Ionisierung von Luft, umfassend mindestens einen Ionenemitter (12), und mindestens einen Emitterträger (14), wobei der mindestens eine Emitterträger (14) ein Profil (16) mit einem Profilquerschnitt (17), einer senkrecht zu dem Profilquerschnitt (17) angeordneten Formlinie (18) und einer senkrecht zur Formlinie (18) angeordneten, das Profil (16) schneidenden Profillängsachse (20) aufweist, der mindestens eine Ionenemitter (12) an dem mindestens einen Emitterträger (14) angeordnet ist, und wobei das Profil (16) als Laminarprofil ausgebildet ist und/oder der Profilquerschnitt (17) als Tragflächenquerschnitt (21) ausgebildet ist,
sowie
Strömungskanal (30) mit einer solchen Vorrichtung (10).

## Beschreibung

Die Erfindung betrifft eine Vorrichtung zur Ionisierung von Luft sowie einen Strömungskanal.

Ionisierung von Luft, insbesondere die Anreicherung von Luft mit Ionen beider Polaritäten, ist ein hinreichend bekanntes Verfahren zur Reinigung und/oder Reinhaltung von Luft, insbesondere zur Reduktion von Keimen oder der Reduzierung von Gerüchen. In einem elektrischen Feld werden dabei Ionen und freie Radikale erzeugt welche Keime wie Bakterien, Viren, Geruchsstoffe usw. deaktivieren. Ein Gerät, mit dem eine solche Luftionisierung erzeugt werden kann und eine spezielle Ausgestaltung der Emitter, also der Elektroden, zwischen denen das elektrische Feld aufgebaut wird, wird beispielsweise in der US 7,254,006 B2 beschrieben.

Bekanntermaßen wird zur Reinigung und/oder Reinhaltung der Luft in einem gegebenen Raumvolumen, unabhängig von der Frage, ob diese durch Filterung oder eine Luftionisierung erfolgt, zweckmäßigerweise ein Luftstrom erzeugt, mit dem gereinigte Luft in das Raumvolumen eingebracht wird während an anderer Stelle Luft aus dem Raumvolumen austritt oder abgesaugt wird und anschließend entweder in die Umgebung abgegeben oder zurückgeführt, gereinigt und wieder in das Raumvolumen eingebracht wird. Dieser Luftstrom wird dabei regelmäßig in einem Strömungskanal erzeugt, der in vielen Anwendungsfällen durch ein Rohr gebildet wird, dessen Querschnitt oft rund ist, aber auch rechteckig sein kann oder eine andere Form aufweist.

Offensichtlich müssen bei Verwendung des Prinzips der Ionisierung von Luft zur Luftreinigung in einem solchen System die Emitter bzw. Elektroden in den Luftstrom eingebracht werden.

Eine Möglichkeit, dies zu realisieren besteht darin, dass das gesamte Gerät zur Luftionisierung komplett mitsamt dem Emitter im Strömungskanal sitzt. Zur Reduzierung des Luftwiderstandes ist es jedoch oft sinnvoll, dass sich nur die Ionenemitter und nicht das gesamte System samt Ansteuerung im Luftstrom befinden. Solche Systeme sind beispielsweise in der DE 10 2008 049 280 A1, der DE 103 11 255 A1 oder der deutschen Patentanmeldung mit dem Aktenzeichen 10 2022 104 843.8 beschrieben.

Oft sollen Geräte zur Luftionisierung in bestehende Lüftungsanlagen nachgerüstet werden. Eine solche Nachrüstlösung ist beispielsweise das *"*Airborne air & surface purification system" des Unternehmens Aviation Clean Air. Bei der Nachrüstung wird ein System aus einem Strömungskanal und einem Gerät zur Luftionisierung gebildet. Das Unterehmen Filt Air Ltd. bietet mit seinem System *"*Sterionizer*"* ebenfalls eine Ionisierungseinheit zum Einbau in einen Lüftungskanal an.

Nachteilig an den bislang bekannten Systemen ist jedoch, dass insbesondere durch die in dem Strömungskanal angeordneten Geräte bzw. deren Emitter Turbulenzen in die durch den Strömungskanal strömende Luft eingebracht werden. Dies begünstigt eine Rekombination der erzeugten Ionen sowie deren Kontakt mit einer Wandung des Strömungskanals, wodurch die Menge der tatsächlich aus dem Strömungskanal austretenden Ionen deutlich reduziert wird. Speziell in Strömungskanälen mit kleineren Querschnitten wird durch herkömmliche Systeme der Strömungswiderstand wesentlich erhöht, was zu einer teils erheblichen Reduzierung des Luftvolumenstroms führt.

Nachteilig an den bekannten Systemen ist darüber hinaus, dass der Emitter, insbesondere die Elektroden, mit der Zeit durch in der Luft befindliche Schmutzpartikel verschmutzen, was zu einer Einschränkung der Effektivität führt. Zur Lösung dieses Problems schlägt die US 8,724,286 B2 eine Ionisierungsvorrichtung mit einem System zur mechanischen Reinigung der Elektroden vor, das in der Herstellung jedoch aufwändig und im Betrieb wartungsintensiv ist.

Der Erfindung liegt daher die Aufgabe zugrunde, eine Anordnung bereitzustellen, mit der eine effektive und gleichzeitig effiziente Ionisierung der Luft möglich ist, die insbesondere die Luftströmung in Strömungskanälen mit geringen Querschnittsflächen nur unwesentlich beeinflusst, und die einen geringen Herstellungs- sowie Wartungsaufwand aufweist.

Die Aufgabe wird erfindungsgemäß gelöst durch eine Vorrichtung mit den Merkmalen des Patentanspruchs 1, eine Vorrichtung mit den Merkmalen des Patentanspruchs 2, sowie einen Strömungskanal mit den Merkmalen des Patentanspruchs 13.

Vorteilhafte Ausgestaltungen und Weiterbildungen der Erfindung sind in den abhängigen Ansprüchen angegeben.

Eine erfindungsgemäße Vorrichtung zur Ionisierung von Luft, umfasst mindestens einen Ionenemitter sowie mindestens einen Emitterträger. Dabei weist der mindestens eine Emitterträger ein Profil mit einem Profilquerschnitt, eine senkrecht zu dem Profilquerschnitt angeordnete Formlinie und eine senkrecht zur Formlinie angeordnete, das Profil schneidende Profillängsachse auf. Der mindestens eine Ionenemitter ist dabei an dem mindestens einen Emitterträger angeordnet. Das Profil kann als Laminarprofil ausgebildet sein. Das Laminarprofil, insbesondere der Profilquerschnitt des Laminarprofils, ist vorzugsweise derart ausgebildet, dass sich bei dessen Um- und/oder Durchströmung, insbesondere mit Luft, in dem Strömungsmedium eine, vorzugsweise ausschließlich, laminare Strömung ausbildet. Stromabwärts des Laminarprofils, insbesondere des Profilquerschnitts des Laminarprofils, ist das Strömungsmedium damit vorzugsweise frei von Turbulenzen. Besonders bevorzugt ist das Laminarprofil, insbesondere der Profilquerschnitt des Laminarprofils, derart ausgebildet, dass sich die laminare Strömung auch bei hohen Strömungsgeschwindigkeiten einstellt. Damit kommen vor allem solche Profilquerschnitte in Betracht, die besonders stromlinienförmig ausgebildet sind, so beispielsweise eine typische Tropfenform. Durch das Vorsehen des Laminarprofils kann die Vorrichtung daher einen geringen Strömungswiderstand aufweisen. Außerdem können so Verwirbelungen stromabwärts des mindestens einen Emitters vermieden und damit eine effektivere Ionisierung erreicht werden. Die Profillängsachse ist vorzugsweise entlang einer für das Profil vorgesehenen Strömungsrichtung angeordnet.

In einer Ausführungsform der Erfindung ist der Profilquerschnitt als Tragflächenquerschnitt ausgebildet. Unter dem Tragflächenquerschnitt wird hier und im Folgenden vorzugsweise ein Querschnitt verstanden, der die typischerweise bei Tragflächen, insbesondere von Flugzeugen, verwendete Form aufweist. Insbesondere kann der Profilquerschnitt damit eine Tropfenform aufweisen. Durch die Ausbildung des Profilquerschnitts als Tragflächenquerschnitt kann der Druckverlust bei der Um- bzw. Durchströmung der Vorrichtung verringert werden.

Der mindestens eine Ionenemitter ist bevorzugt durch zwei, besonders bevorzugt nadelförmige, Elektroden gebildet. Dabei können beide Elektroden an demselben mindestens einen Emitterträger oder an unterschiedlichen Emitterträgern angeordnet sein. Insbesondere nadelförmige Elektroden weisen typischerweise einen geringen Strömungswiderstand auf und können leicht herstellbar und montierbar ausgebildet werden.

Der mindestens eine Ionenemitter ist vorzugsweise an dem Profil angeordnet. Die Vorrichtung kann so als eine räumlich zusammenhängende Baugruppe ausgebildet sein. Insbesondere die Integration und Montage der Vorrichtung in einen bestehenden Strömungskanal, wie eine Rohrleitung, kann dadurch erleichtert werden.

Das Profil kann eine Ausnehmung aufweisen, in der der mindestens eine Ionenemitter angeordnet ist. Vorzugsweise ist die Ausnehmung an einer der vorgesehenen Strömungsrichtung abgewandten Fläche angeordnet. Sofern der Profilquerschnitt als Tragflächenquerschnitt ausgebildet ist, ist die Ausnehmung vorzugsweise an einer hinteren Kante des Profils angeordnet. Die Ausnehmung kann eine Ausnehmungskontur aufweisen, die insbesondere halbkreis- oder V-förmig oder mehreckig, insbesondere rechteckig ausgebildet sein kann. Die Ausnehmungskontur kann, zumindest abschnittsweise, auch einer Kurve zweiter Ordnung, insbesondere einer Parabel, oder höherer Ordnung entsprechen. Durch die Anordnung des mindestens einen Ionenemitters in der Ausnehmung kann die Länge der Vorrichtung entlang der Profillängsachse reduziert werden. Dadurch kann der Einbau der Vorrichtung insbesondere in einen bestehenden Strömungskanal vereinfacht werden. Jede der Elektroden des mindestens einen Emitters ist vorzugsweise in einem der beiden den beiden Endbereichen der Ausnehmung angeordnet. Besonders bevorzugt sind die beiden Elektroden damit in unterschiedlichen Endbereichen der Ausnehmung angeordnet. Ein üblicher Abstand der Elektroden zueinander beträgt dabei ca. 20 mm.

Die Ausnehmung kann das Profil senkrecht zu einer von der Profillängsachse und der Formlinie aufgespannten Fläche vollständig schneiden. So können die Elektroden bezogen auf diese Fläche beidseitig angeströmt werden, wodurch die Effektivität der Vorrichtung verbessert werden kann.

An und/oder in dem mindestens einen Emitterträger, insbesondere dem Profil, kann eine Leiterplatte zur Spannungsversorgung des mindestens einen Ionenemitters angeordnet sein. Dadurch kann die Installation der Spannungsversorgung an und/oder in dem mindestens einen Emitterträger vereinfacht werden. So kann das verhältnismäßig aufwändige Verlegen einzelner Kabel an und/oder in dem mindestens einen Emitterträger durch das Anordnen der Leiterplatte ersetzt werden. Zum Anordnen der Leiterplatte an und/oder in dem mindestens einen Emitterträger kann zwischen der Leiterplatte und dem mindestens einen Emitterträger eine Steck- und/oder Clipverbindung vorgesehen sein. Die Leiterplatte kann mehrere Lagen aufweisen. Beispielsweise kann die Leiterplatte zweilagig oder vierlagig ausgeführt sein. Unterschiedliche elektrische Potentiale werden dabei vorzugsweise auf unterschiedlichen Lagen der Leiterplatte geführt. So kann ein Überspringen der Spannung bei den relativ hohen an dem mindestens einen Ionenemitter anliegenden Spannungen vermieden werden. In einer einfachen Ausführungsform kann die Leiterplatte durch zwei Leiter, beispielsweise Bleche, mit einer zwischen den Leitern angeordneten Isolationsschicht ausgebildet sein.

Vorzugsweise ist der mindestens eine Ionenemitter, insbesondere unmittelbar, auf der Leiterplatte angeordnet. So kann jede der Elektroden, insbesondere unmittelbar, an jeweils einer auf der Leiterplatte angeordneten Leiterbahn angeordnet werden. Eine derartige Anordnung kann insbesondere einfach automatisiert herstellbar sein, typischerweise in dem die Leiterplatte automatisiert mit den Elektroden bestückt wird.

In einer Weiterbildung der Erfindung weist das Profil an mindestens einer die Formlinie schneidenden Stirnseite einen, insbesondere elektrischen, Steckverbinder auf. So kann das Profil, und damit der an und/oder in dem Profil angeordnete mindestens eine Emitterträger einfach und wiederholbar mit einer Spannungsversorgung verbunden werden. Dadurch kann die Installation der Vorrichtung in einem Strömungskanal vereinfacht werden. Bei der Installation kann der mindestens eine Emitterträger beispielsweise über eine seitlich in dem Strömungskanal angeordnete Öffnung in den Strömungskanal eingeführt werden. Der Steckverbinder erlaubt ein einfaches Verbinden des mindestens einen Emitterträgers mit einem Anschlusselement bei oder nach dem Anordnen des mindestens einen Emitterträgers in dem Strömungskanal. Der Steckverbinder kann verriegelbar ausgebildet sein. Die Stirnseite kann parallel zu der Profillängsachse angeordnet sein.

Die Formlinie kann gerade, kreisförmig oder oval ausgebildet sein. Die Formgebung des Profils kann damit so gewählt werden, dass sie an die von dem Strömungskanal vorgegebenen Randbedingungen, insbesondere eine Querschnittsform und/oder eine Querschnittsfläche des Strömungskanalquerschnitts, angepasst werden kann. So kann ein kreisringförmiges Profil beispielsweise eine kreisförmige Formlinie aufweisen. Insbesondere ein Profil mit einer geraden Formlinie kann besonders einfach in einen bestehenden Strömungskanal nachrüstbar sein, da es sich in der zuvor beschriebenen Weise einfach durch eine seitliche Öffnung in den Strömungskanal einführen lässt.

In einer Ausführungsform der Erfindung umfasst der mindestens eine Emitterträger eine Mehrzahl von Emitterträgern. Dadurch kann die Vorrichtung derart ausgestaltet werden, dass sie vor allem in Strömungskanälen mit größerer Querschnittsfläche, insbesondere mit einem Durchmesser von 150 mm oder mehr, eine effektive Ionisierung der Luft ermöglicht. Die Emitterträger können dabei einen winkligen und/oder linearen Versatz zueinander aufweisen.

Die Emitterträger können beispielsweise sternförmig, insbesondere um eine zentrale Anschlussleitung, oder parallel zueinander angeordnet sein. Bei einer parallelen Anordnung weisen die Emitterträger vorzugsweise einen ausschließlich parallelen Versatz zueinander auf. Dabei sind vorzugsweise die Profillängsachsen und die, bevorzugt gerade ausgebildeten, Formlinien der Emitterträger jeweils parallel zueinander angeordnet. Diese Anordnung bietet sich insbesondere für rechteckige Strömungskanalquerschnitte mit größerer Querschnittsfläche an. Bei einer sternförmigen Anordnung sind die Emitterträger vorzugsweise derart angeordnet, dass die Profillängsachsen der Emitterträger parallel zueinander und die, vorzugsweise geraden, Formlinien mit einem winkligen Versatz zueinander angeordnet sind. Die Anschlussleitung umfasst insbesondere die Spannungsversorgung der an den Emitterträgern angeordneten Ionenemitter.

Ein erfindungsgemäßer Strömungskanal umfasst eine Kanallängsachse, eine Strömungsrichtung entlang der Kanallängsachse und eine zuvor beschriebene Vorrichtung zur Ionisierung von Luft. Dabei ist das Profil der Vorrichtung derart in dem Strömungskanal angeordnet, dass die Formlinie senkrecht zu der Kanallängsachse angeordnet ist. Die Profillängsachse kann parallel zu der Kanallängsachse angeordnet sein. Dadurch kann der Emitterträger, und insbesondere das Profil, einen möglichst geringen Strömungswiderstand hervorrufen. Alternativ können die Profillängsachse und die Kanallängsachse einen Anstellwinkel von größer als 0° einschließen. Insbesondere bei einer sternförmigen Anordnung der Emitterträger kann dadurch ein Drall in dem Luftstrom erzeugt werden. Der Anstellwinkel kann dabei veränderbar und damit einstellbar ausgebildet sein. Mit einem derartigen mindestens einen Emitterträger kann der Luftstrom in dem Strömungskanal reguliert werden. Der mindestens eine Emitterträger kann dabei nach Art einer Drosselklappe ausgebildet sein. Mit einem Anstellwinkel von ungefähr 90° kann der Luftstrom damit maximal gedrosselt werden. Dabei kann der mindestens eine Emitterträger derart ausgebildet sein, dass der Strömungskanalquerschnitt zumindest näherungsweise vollständig geschlossen werden kann.

Zur Montage des erfindungsgemäßen Strömungskanals kann der mindestens eine Emitterträger, wie zuvor beschrieben, durch eine seitlich in dem Strömungskanal angeordnete Öffnung in den Strömungskanal eingeführt werden. Dieses Vorgehen bietet sich insbesondere bei Strömungskanälen an, die nur einen Emitterträger und/oder ausschließlich Emitterträger mit einer geraden Formlinie aufweisen. Alternativ kann die Vorrichtung in einem Strömungskanalabschnitt angeordnet sein, der entlang der Kanallängsachse in den Strömungskanal einsetzbar ist. Diese Lösung bietet sich insbesondere bei Strömungskanälen an, die mehrere Emitterträger und/oder Emitterträger mit einer gekrümmten Formlinie aufweisen. Der erfindungsgemäße Strömungskanal kann als allgemeiner Strömungskanal ausgebildet sein. In einer Weiterbildung der Erfindung kann der Strömungskanal einen Luftauslass, insbesondere einen Deckenauslass, umfassen. Ein derartiger Luft- bzw. Deckenauslass ist vorzugsweise an einem Ende des Strömungskanals angeordnet, der insbesondere in einen Raum mündet. Ein als Deckenauslass ausgebildeter Luftauslass ist vorzugsweise an einer Decke des Raums angeordnet. Besonders bevorzugt ist die Vorrichtung dabei in dem Luftauslass oder in Strömungsrichtung unmittelbar vor dem Luftauslass angeordnet.

Der mindestens eine Ionenemitter ist vorzugsweise in einem laminar umströmten Bereich des mindestens einen Emitterträgers angeordnet. Dadurch kann die Rekombinationswahrscheinlichkeit der mittels des mindestens einen Ionenemitters erzeugten Ionen verringert und damit die Effizienz gesteigert werden.

Vorzugsweise ist der mindestens eine Ionenemitter an einer zumindest teilweise in der Strömungsrichtung ausgerichteten Fläche des Profils angeordnet. Damit kann ein Kollidieren der erzeugten Ionen mit dem Profil selbst vermieden werden. Unter der zumindest teilweise in der Strömungsrichtung ausgerichteten Fläche wird vorzugsweise eine Fläche des Profils verstanden, die in die Strömungsrichtung geneigt ist. Ein Neigungswinkel zwischen der entsprechenden Fläche und der Strömungsrichtung liegt vorzugsweise im Bereich von 0° bis kleiner 90°. Die entsprechende Fläche ist bezogen auf die Strömungsrichtung damit vorzugsweise auf einer Lee-Seite des Profils angeordnet.

Besonders bevorzugt ist der mindestens eine Ionenemitter in der Strömungsrichtung an einer hinteren Kante des Tragflächenquerschnitts angeordnet. Der Begriff der "hinteren Kante" bezieht sich dabei vorzugsweise auf die Strömungsrichtung. Die Elektroden sind vorzugsweise in der Strömungsrichtung ausgerichtet.

Mehrere Ausführungsbeispiele der Erfindung werden anhand der nachfolgenden Figuren erläutert. Es zeigt:
- Figur 1: ein erstes Ausführungsbeispiel einer Vorrichtung zur Ionisierung von Luft,
- Figur 2: ein zweites Ausführungsbeispiel einer Vorrichtung zur Ionisierung von Luft,
- Figur 3: ein erstes Ausführungsbeispiel eines Strömungskanals mit einer parallelen Anordnung mehrerer des in Fig. 1 gezeigten Emitterträgers,
- Figur 4: ein zweites Ausführungsbeispiel eines Strömungskanals mit einer sternförmigen Anordnung mehrerer des in Fig. 1 gezeigten Emitterträgers,
- Figur 5: ein drittes Ausführungsbeispiel eines Strömungskanals mit einer sternförmigen Anordnung mehrerer des in Fig. 1 gezeigten Emitterträgers,
- Figur 6: das in Fig. 5 gezeigte Ausführungsbeispiel mit angestellten Profilen,
- Figur 7: ein viertes Ausführungsbeispiel eines Strömungskanals mit einer parallelen Anordnung der Emitterträger,
- Figur 8: ein fünftes Ausführungsbeispiel eines Strömungskanals mit einem kreisringförmigen Profil.

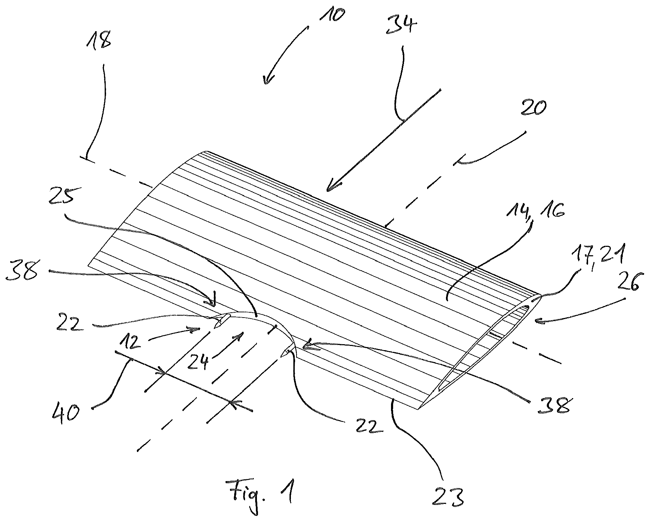

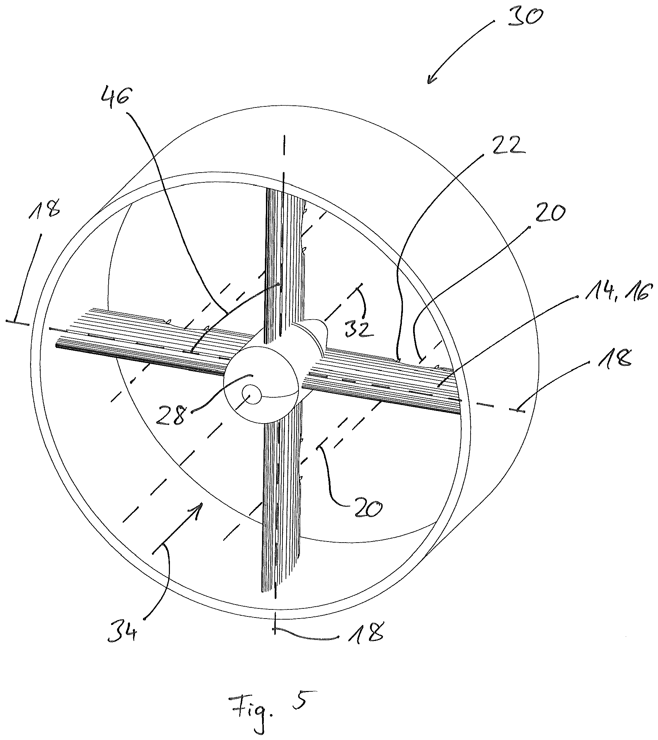

Die Figuren 1 bis 8 zeigen verschiedene Ansichten verschiedener Ausführungsbeispiele. Der Übersichtlichkeit halber werden nicht alle Bezugszeichen in jeder Figur verwendet. Für gleiche und funktionsgleiche Teile werden die gleichen Bezugszeichen verwendet.

Fig. 1 u. 2 zeigen jeweils ein Ausführungsbeispiel einer Vorrichtung 10 zur Ionisierung von Luft mit einem Ionenemitter 12 sowie einem Emitterträger 14. Dabei ist der Emitterträger 14 als Profil 16 ausgebildet. Ein Profilquerschnitt 17 des Profils 16 ist an einer Stirnseite 26 des Profils 16 erkennbar. Senkrecht zu dem Profilquerschnitt 17 ist eine Formlinie 18 angeordnet. Das Profil 16 weist ferner eine senkrecht zur Formlinie 18 angeordnete, das Profil 16 schneidende Profillängsachse 20 auf. Die Stirnseite 26 ist vorzugsweise parallel zu der Profillängsachse 20 angeordnet.

Das Profil 16 ist vorzugsweise als Laminarprofil ausgebildet, dessen Profilquerschnitt 17 derart ausgebildet ist, dass sich bei dessen Umströmung mit Luft in einer vorgesehenen Strömungsrichtung 34 eine, vorzugsweise ausschließlich, laminare Strömung ausbildet. Die Profillängsachse 20 ist entlang der für das Profil 16 vorgesehenen Strömungsrichtung 34 angeordnet. Entlang der Profillängsachse 20 ist das Profil 16 dementsprechend besonders stromlinienförmig ausgebildet. Dafür weist der Profilquerschnitt 17 eine typische Tropfenform auf. Wie aus Fig. 1 u. 2, insbesondere an der Stirnseite 26 des jeweiligen Profils 16, erkennbar ist, ist in diesen Ausführungsbeispielen der Vorrichtung 10 der Profilquerschnitt 17 als Tragflächenquerschnitt 21 ausgebildet, wie er beispielsweise bei den Tragflächen von Flugzeugen Verwendung findet.

Der Ionenemitter 12 ist durch zwei nadelförmige Elektroden 22 gebildet, die an demselben Emitterträger 14 angeordnet sind. Die nadelförmigen Elektroden 22 weisen dabei einen geringen Strömungswiderstand auf und können leicht herstellbar und montierbar ausgebildet werden. Bezogen auf die vorgesehene Strömungsrichtung 34 ist der Ionenemitter 12 an einer hinteren Kante 23 des Tragflächenquerschnitts 21 angeordnet. Die Elektroden 22 sind in der vorgesehenen Strömungsrichtung 34 ausgerichtet. Der Ionenemitter 12 ist dabei an dem Profil 10 angeordnet, sodass die Vorrichtung 10 als eine räumlich zusammenhängende Baugruppe ausgebildet ist. Insbesondere die Integration und Montage der Vorrichtung 10 kann dadurch erleichtert werden.

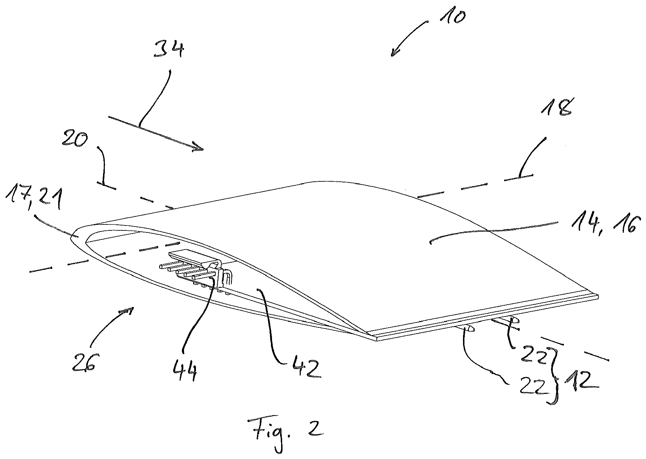

Im Unterschied zu dem Ausführungsbeispiel der Fig. 2, weist das in Fig. 1 gezeigte Profil 16 eine Ausnehmung 24 auf, in der Ionenemitter 12 angeordnet ist. Dabei ist die Ausnehmung 24 an der hinteren Kante 23 des Profils 16 angeordnet, und weist eine parabelförmige Ausnehmungskontur 25 auf. In Fig. 1 ist gut erkennbar, dass durch die Anordnung des Ionenemitters 12 in der Ausnehmung 24 die Länge der Vorrichtung 10 entlang der Profillängsachse 20 reduziert werden kann. Dadurch kann der Einbau der Vorrichtung 10 insbesondere in einen bestehenden Strömungskanal 30 vereinfacht werden. Wie Fig. 1 außerdem zeigt, sind die beiden Elektroden 22 des Ionenemitters 12 in unterschiedlichen Endbereichen 38 der Ausnehmung 24 angeordnet. Ein üblicher Abstand 40 der Elektroden zueinander beträgt dabei ca. 20 mm. Die Ausnehmung 24 schneidet das Profil 16 senkrecht zu einer von der Profillängsachse 20 und der Formlinie 18 aufgespannten Fläche vollständig, sodass die Elektroden 22 bezogen auf diese Fläche beidseitig angeströmt werden können.

Aus dem in Fig. 2 gezeigten Ausführungsbeispiel ist ersichtlich, dass in dem Emitterträger 14, insbesondere in dem Profil 16, eine Leiterplatte 42 zur Spannungsversorgung des Ionenemitters 12 angeordnet ist. Dadurch kann die Installation der Spannungsversorgung in dem Emitterträger 14 vereinfacht werden, vor allem weil so das verhältnismäßig aufwändige Verlegen einzelner Kabel an und/oder in dem Emitterträger 14 durch das Anordnen der Leiterplatte 42 ersetzt werden kann. Zum Anordnen der Leiterplatte 42 in dem Emitterträger 14 kann zwischen der Leiterplatte 42 und dem Emitterträger 14 eine in Fig. 2 nicht dargestellte Steck- und/oder Clipverbindung vorgesehen sein. Die Leiterplatte 42 kann mehrere Lagen aufweisen. Unterschiedliche elektrische Potentiale werden dabei vorzugsweise auf unterschiedlichen Lagen der Leiterplatte 42 geführt. So kann ein Überspringen der Spannung bei den relativ hohen an dem Ionenemitter 12 anliegenden Spannungen vermieden werden.

Wie Fig. 2 ferner zeigt, ist der Ionenemitter 12 unmittelbar auf der Leiterplatte 42 angeordnet. Vorzugsweise ist dabei jede der Elektroden 22 an jeweils einer auf der Leiterplatte 42 angeordneten Leiterbahn angeordnet. Zur Herstellung dieser Anordnung wird die Leiterplatte 42 typischerweise automatisiert mit den Elektroden 22 bestückt.

In Fig. 2 ist außerdem dargestellt, dass das Profil 16 an mindestens einer der die Formlinie 18 schneidenden Stirnseite 26 einen elektrischen Steckverbinder 44 aufweist. Der Steckverbinder 44 erlaubt ein einfaches Verbinden des Emitterträgers 14 mit einem Anschlusselement bei oder nach dem Anordnen des Emitterträgers 14 in dem Strömungskanal 30. Der Steckverbinder 44 kann verriegelbar ausgebildet sein.

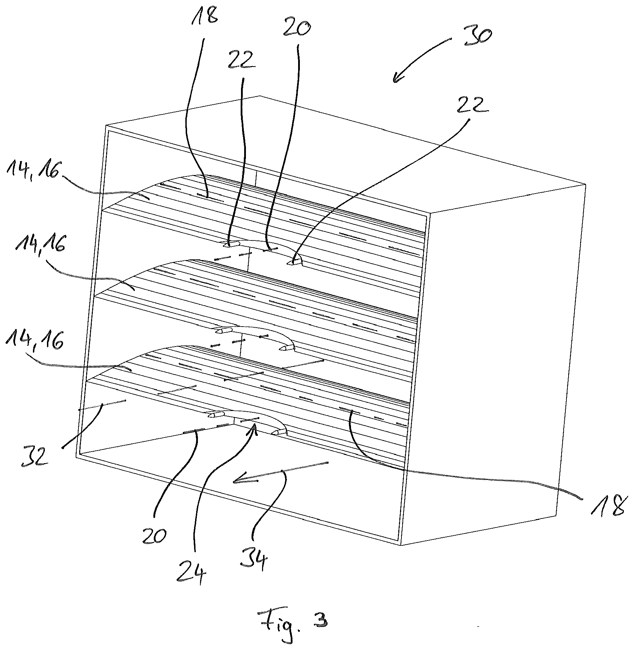

Die Fig. 3-8 zeigen verschiedene Ausführungsbeispiele des Strömungskanals 30, in denen unterschiedliche Ausführungsbeispiele der Vorrichtung 10 zur Ionisierung von Luft angeordnet sind. Der Strömungskanal 30 jedes der Ausführungsbeispiele weist eine Kanallängsachse 32 auf. Die Strömungsrichtung 34 des jeweiligen Strömungskanals 30 ist entlang der Kanallängsachse 32 ausgerichtet. Die Vorrichtungen 10 sind derart in dem jeweiligen Strömungskanal 30 angeordnet, dass die in dem jeweiligen Strömungskanal vorliegende Strömungsrichtung 34 mit der in den Fig. 1 eingezeichneten, vorgesehenen Strömungsrichtung 34 übereinstimmt. In jedem der Ausführungsbeispiele der Fig. 3 bis 8 ist das Profil 16 des mindestens einen Emitterträgers 14 derart in dem Strömungskanal 30 angeordnet, dass die jeweilige Formlinie 18 senkrecht zu der Kanallängsachse 32 angeordnet ist. In den Ausführungsbeispielen der Fig. 3-5 sind außerdem jeweils die Profillängsachse 20 parallel zu der Kanallängsachse 32 angeordnet.

Bei den in Fig. 3-6 gezeigten Ausführungsbeispielen des Strömungskanals 30 sind die Emitterträger 14 entsprechend dem Emitterträger 14 der in Fig. 1 gezeigten Vorrichtung ausgebildet. Das in Fig. 3 gezeigte Ausführungsbeispiel weist drei der Emitterträger 14 auf. Jeder der Emitterträger 14 kann dabei mehr als ein Paar der Elektroden 22 und damit mehrere Ionenemitter 12 aufweisen. Wie Fig. 3 zeigt, sind die Emitterträger 14 in diesem Ausführungsbeispiel parallel zueinander angeordnet. Dementsprechend sind die Profillängsachsen 20 und die - gerade ausgebildeten - Formlinien der Emitterträger 14 jeweils parallel zueinander angeordnet. Dementsprechend weist der in Fig. 3 gezeigte Strömungskanal 30 einen rechteckigen Strömungskanalquerschnitt mit verhältnismäßig großer Querschnittsfläche auf.

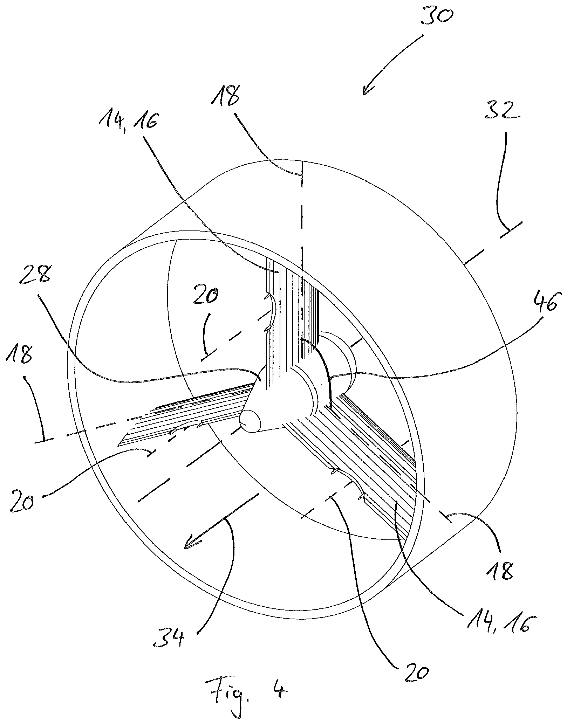

Die Ausführungsbeispiele der Fig. 4-6 zeigen eine alternative Anordnung, bei der die Emitterträger 14 sternförmig um eine zentrale Anschlussleitung 28 angeordnet sein. Dabei sind die Emitterträger 14 vorzugsweise derart angeordnet, dass die Profillängsachsen 20 der Emitterträger 14 parallel zueinander und die - vorzugsweise geraden - Formlinien 18 mit einem winkligen Versatz 46 zueinander angeordnet sind. Die Anschlussleitung 28 umfasst insbesondere die Spannungsversorgung der an den Emitterträgern 14 angeordneten Ionenemitter 12. Vorzugsweise ist die Vorrichtung 10 in einem Strömungskanalabschnitt angeordnet, der zur Montage entlang der Kanallängsachse 32 in den Strömungskanal 30 einsetzbar ist. Wie die Ausführungsbeispiele der Fig. 4 u. 5 zeigen, kann die in dem Strömungskanal 30 angeordnete Vorrichtung 10 beispielsweise drei oder vier Emitterträger 14 aufweisen.

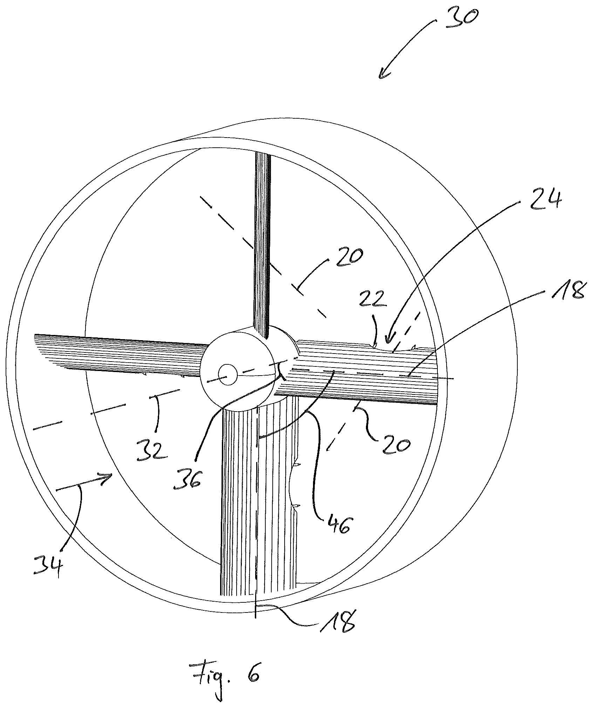

Fig. 6 zeigt ein Ausführungsbeispiel, bei dem die Profillängsachsen 20 und die Kanallängsachse 32 jeweils einen Anstellwinkel 36 von größer als 0° einschließen. Insbesondere im Zusammenspiel mit der sternförmigen Anordnung der Emitterträger 14 kann dadurch ein Drall in dem Luftstrom erzeugt werden. Der Anstellwinkel 36 kann veränderbar und damit einstellbar ausgebildet sein. Mit einer derartigen Anordnung der Emitterträger 14 kann der Luftstrom in dem Strömungskanal 30 zumindest teilweise reguliert werden.

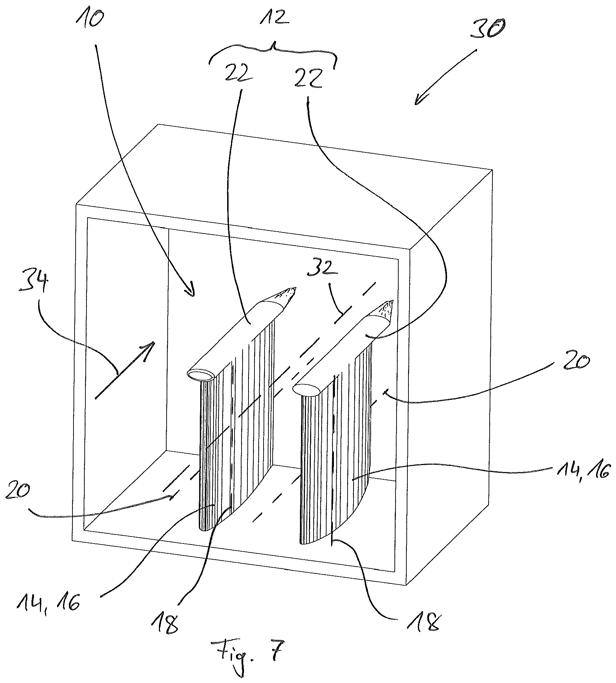

Das Ausführungsbeispiel der Fig. 7 weist zwei parallel zueinander angeordnete Emitterträger 14 auf. Im Unterschied zu dem in Fig. 3 gezeigten Ausführungsbeispiel sind diese Emitterträger 14 jedoch nicht entsprechend der Darstellung in Fig. 1 ausgebildet. Vielmehr weisen die in Fig. 7 gezeigten Emitterträger 14 keine Ausnehmung 24 auf. Außerdem sind die beiden Elektroden 22 des Ionenemitters 12 an unterschiedlichen Emitterträger 14 angeordnet. Eine derartige Lösung kann sich insbesondere anbieten, wenn der Strömungskanal 30 einen rechteckigen Strömungskanalquerschnitt jedoch eine kleinere Querschnittsfläche als der in Fig. 3 gezeigte Strömungskanal 30 aufweist. Zur Montage dieses Strömungskanals 30 können die Emitterträger 14 beispielsweise durch eine seitlich in dem Strömungskanal 30 angeordnete Öffnung in den Strömungskanal 30 eingeführt werden.

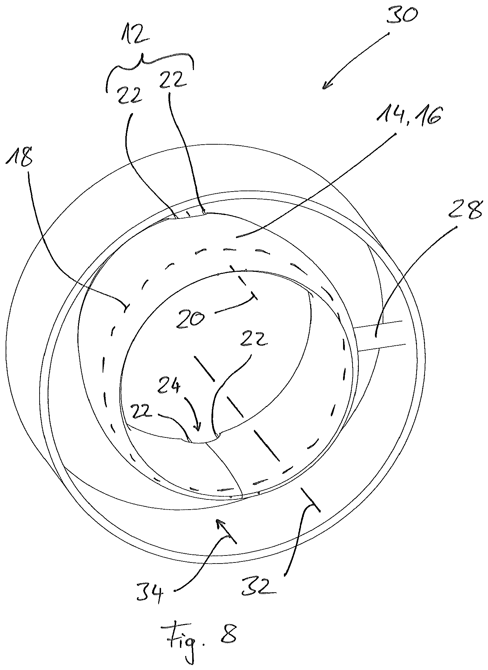

Während die in den Fig. 1-7 gezeigten Profile 16 jeweils eine gerade Formlinie 18 aufweisen, zeigt das Ausführungsbeispiel der Fig. 8, dass die Formlinie 18 gekrümmt und insbesondere kreisförmig ausgebildet sein kann. Die Formgebung des Profils 16 kann damit insbesondere an einen kreisförmigen Strömungskanalquerschnitt mit einer verhältnismäßig großen Querschnittsfläche angepasst werden. Das in Fig. 8 gezeigte Profil 16 kann ebenfalls den in den übrigen Figuren gezeigten Tragflächenquerschnitt 21 aufweisen. Die Vorrichtung 10 des in Fig. 8 gezeigten Ausführungsbeispiels kann darüber hinaus zwei oder mehr Ionenemitter 12 aufweisen. Die Anschlussleitung 28 ist seitlich angeordnet.

### Bezugszeichenliste

- 10: Vorrichtung
- 12: Ionenemitter
- 14: Emitterträger
- 16: Profil
- 17: Profilquerschnitt
- 18: Formlinie
- 20: Profillängsachse
- 21: Tragflächenquerschnitt
- 22: Elektrode
- 23: hintere Kante
- 24: Ausnehmung
- 25: Ausnehmungskontur
- 26: Stirnseite
- 28: Anschlussleitung
- 30: Strömungskanal
- 32: Kanallängsachse
- 34: Strömungsrichtung
- 36: Anstellwinkel
- 38: Endbereich
- 40: Abstand
- 42: Leiterplatte
- 44: Steckverbinder
- 46: winkliger Versatz

## Patentansprüche

1. Vorrichtung (10) zur Ionisierung von Luft, umfassend:
• mindestens einen Ionenemitter (12),
• mindestens einen Emitterträger (14),
wobei
• der mindestens eine Emitterträger (14) ein Profil (16) mit einem Profilquerschnitt (17), einer senkrecht zu dem Profilquerschnitt (17) angeordneten Formlinie (18) und einer senkrecht zur Formlinie (18) angeordneten, das Profil (16) schneidenden Profillängsachse (20) aufweist, und
• der mindestens eine Ionenemitter (12) an dem mindestens einen Emitterträger (14) angeordnet ist,
**dadurch gekennzeichnet, dass** das Profil (16) als Laminarprofil ausgebildet ist.

2. Vorrichtung nach dem Oberbegriff des Anspruchs 1 oder Anspruch 1,
**dadurch gekennzeichnet, dass**
der Profilquerschnitt (17) als Tragflächenquerschnitt (21) ausgebildet ist.

3. Vorrichtung nach einem der vorhergehenden Ansprüche,
**dadurch gekennzeichnet, dass**
der mindestens eine Ionenemitter (12) durch zwei, vorzugsweise nadelförmige, Elektroden (22) gebildet ist.

4. Vorrichtung nach einem der vorhergehenden Ansprüche,
**dadurch gekennzeichnet, dass**
der mindestens eine Ionenemitter (12) an dem Profil (16) angeordnet ist.

5. Vorrichtung nach einem der vorhergehenden Ansprüche,
**dadurch gekennzeichnet, dass**
das Profil (16) eine Ausnehmung (24) aufweist, in der der mindestens eine Ionenemitter (12) angeordnet ist.

6. Vorrichtung nach Anspruch 5,
**dadurch gekennzeichnet, dass**
die Ausnehmung (24) das Profil (16) senkrecht zu einer von der Profillängsachse (20) und der Formlinie (18) aufgespannten Fläche vollständig schneidet.

7. Vorrichtung nach einem der vorhergehenden Ansprüche,
**dadurch gekennzeichnet, dass**
an und/oder in dem mindestens einen Emitterträger (14), insbesondere dem Profil (16), eine Leiterplatte zur Spannungsversorgung des mindestens einen Ionenemitters (12) angeordnet ist.

8. Vorrichtung nach Anspruch 7,
**dadurch gekennzeichnet, dass**
der mindestens eine Ionenemitter (12) auf der Leiterplatte angeordnet ist.

9. Vorrichtung nach einem der vorhergehenden Ansprüche,
**dadurch gekennzeichnet, dass**
das Profil (16) an mindestens einer die Formlinie (18) schneidenden Stirnseite (26) einen, insbesondere elektrischen, Steckverbinder (44) aufweist.

10. Vorrichtung nach einem der vorhergehenden Ansprüche,
**dadurch gekennzeichnet, dass**
die Formlinie (18) gerade, kreisförmig oder oval ausgebildet ist.

11. Vorrichtung nach einem der vorhergehenden Ansprüche,
**dadurch gekennzeichnet, dass**
der mindestens eine Emitterträger (14) eine Mehrzahl von Emitterträgern (14) umfasst.

12. Vorrichtung nach Anspruch 10,
**dadurch gekennzeichnet, dass**
die Emitterträger (14) sternförmig, insbesondere um eine zentrale Anschlussleitung (28), oder parallel zueinander angeordnet sind.

13. Strömungskanal (30) mit einer Kanallängsachse (32), einer Strömungsrichtung (34) entlang der Kanallängsachse (32) und einer Vorrichtung (10) zur Ionisierung von Luft nach einem der vorhergehenden Ansprüche, wobei das Profil (16) der Vorrichtung (10) derart in dem Strömungskanal (30) angeordnet ist, dass die Formlinie (18) senkrecht zu der Kanallängsachse (32) angeordnet ist, und dass die Profillängsachse (20) parallel zu der Kanallängsachse (32) angeordnet ist, oder die Profillängsachse (20) und die Kanallängsachse (32) einen Anstellwinkel (36) von größer als 0° einschließen.

14. Strömungskanal nach Anspruch 13,
**dadurch gekennzeichnet, dass**
der mindestens eine Ionenemitter (12) in einem laminar umströmten Bereich des mindestens einen Emitterträgers (14) angeordnet ist.

15. Strömungskanal nach einem der Ansprüche 13 bis 14,
**dadurch gekennzeichnet, dass**
der mindestens eine Ionenemitter (12) an einer zumindest teilweise in der Strömungsrichtung (24) ausgerichteten Fläche des Profils (16) angeordnet ist.

16. Strömungskanal nach einem der Ansprüche 13 bis 15 mit einer Vorrichtung nach Anspruch 2 oder Anspruch 2 und einem der Ansprüche 3 bis 12,
**dadurch gekennzeichnet, dass**
der mindestens eine Ionenemitter (12) in der Strömungsrichtung (34) an einer hinteren Kante (23) des Tragflächenquerschnitts (21) angeordnet ist.
